# EUROPEAN PATENT APPLICATION

(11) **EP 2 233 071 A1**
(43) Date of publication of application: **29.09.2010**
(21) Application number: 09156452.6
(22) Date of filing: 27.03.2009
(51) Int. Cl.: A61B 5/08, A61B 5/113, A63B 23/18

(54) **Breathing feedback device**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention relates to a breathing feedback device (1) and a method for operating a breathing feedback device for making the breathing pattern of a human user (11) audible for the user, wherein the breathing pattern is detected by a camera (5), a heat-sensitive sensor, a pressure-sensitive sensor, an inertial sensor and/or a heart rate detector.

## Description

### FIELD OF THE INVENTION

The present invention relates to the field of generating audible breathing feedbacks.

### BACKGROUND OF THE INVENTION

Breathing is one of the few bodily functions which, within limits, can be controlled both consciously and unconsciously. It is well known, that correct breathing contributes to health and feelings of well-being, whereby the correct breathing could be influenced by feedback the breathing pattern to the breathing person.

For example, a biofeedback device is disclosed by the US patent application US2007/0173730 Al having a microphone configured to acquire sounds of a user's breathing; a controller communicatively connected with the microphone, the controller processing the signals acquired by the microphone to produce an output signal, the controller processing the signal whereby the microphone signal is first pre-amplified to a voltage level that can be processed by an audio envelope detector circuit, the envelope detector signal is then fed into the analog-to-digital converter input of the controller allowing it to constantly sample the input volume level, the controller then controlling the output volume level fed to the headphones utilizing a digitally controlled variable-gain amplifier, wherein the output signal is not modified in any manner from the original input, except in volume; and a pair of earphones connected with the controller and configured to convey the output signal to the user. Disadvantageously, said biofeedback device is based on acquiring the user's sound merely with the aid of a microphone, whereby the actual breathing pattern is not detected directly by the biofeedback device. This leads to a decreased accuracy of the feedback pattern compared to the actual breathing pattern as the user's sound can strongly derivate from the actual breathing characteristics. Furthermore, the accurate detection of the user's sound could be very difficult or even impossible, if there are further sound sources or other breathing persons near the user. Another drawback of said biofeedback device is the low distance between the user's head and the microphone which is necessary to acquire the user's sound.

### SUMMARY OF THE INVENTION

It is an object of the present invention to provide a breathing feedback device and a method for operating a breathing feedback device which accurately detects the actual breathing pattern of a breathing user and does not have the drawbacks mentioned in connection with the prior art.

The above mentioned object is accomplished by a breathing feedback device comprising:
- a breathing detector for detecting a breathing pattern,
- a processing unit for generating an output signal depending on the detected breathing pattern and
- a sound generator for playing the output signal,
- whereas the breathing detector comprises a camera.

Advantageously, the breathing feedback device according to the present invention uses the aid of a camera to detect the breathing pattern of the user instead of using a microphone. The camera is directed towards the head of the user and in particular to his mouth detecting characteristic breathing patterns. It is an advantage of the present invention over the above mentioned state of the art that other sound noises or the sound of another breathing person do not influence the accuracy of the detection. Furthermore, the implementation and usage of the camera in the breathing detector is simpler, more economical, more compact und first of all more accurately in comparison to a microphone.

In a preferred embodiment of the present invention, the camera comprises an optical camera, a thermographic camera, a radar detection device and/or a photoplethysmograph. Beneficially, said cameras provide accurate detection of the actual breathing pattern without interferences. The optical camera allows the detection of the breathing pattern by observing the motion of the mouth, the lips and/or the thorax of the user. The airflow into and/or out of the mouth/nose of the user during breathing can be observed with the thermographic camera by scanning the infrared spectrum of the airflow. The airflow can be divided into out-flow and in-flow due to differences in temperature and/or moisture of the air. Beneficially, the airflow is inseparably connected with the breathing of the user, so that the usage of a thermographic camera allows a very reliable and precise detection of the breathing pattern. In another preferred embodiment the airflow is observed by a radar detection device to achieve a suchlike high quality detection of the breathing pattern.

Preferably, the breathing pattern is determined by utilizing Respiratory sinus arrhythmia (RSA), wherein the respiration of the user can be recovered by measuring the heart rate of the user. In particular, the heart rate of the user is measured with a Photoplethysmographic Imager (PPGI) which diagnoses the skin perfusion of the user using near-infrared and subsequently determines the heart rate in dependency of the diagnosed skin perfusion. Advantageously, the respiration of the user can therewith be detected by the aid of a camera, even if the user is laying with his mouth on the pillow or if the user is out of sight of the camera.

Another object of the present invention is a breathing feedback device comprising:
- a breathing detector for detecting a breathing pattern,
- a processing unit for generating an output signal depending on the detected breathing pattern and
- a sound generator for playing the output signal,
- whereas the breathing detector comprises a force sensor.

Beneficially, the usage of a force sensor provides the detection of the breathing pattern based on the uniform and continuous movement of the person's lung during breathing which is detectable by measuring mechanical forces and/or inertia forces and/or tension forces and/or pressure as a mechanical force per area. It is an advantage of the present invention that the movement of the lung is directly connected to the actual breathing characteristics of the person, so that the usage of the force sensor allows a very reliable and precise detection of the breathing pattern.

In a preferred embodiment the force sensor comprises an inertial sensor for determining the breathing pattern in dependency of a chest movement, whereby the inertial sensor is attached to the chest of the person for instance and measures the movement of the chest caused by the movement of the person's lung. In another preferred embodiment of the present invention the force sensor comprises a strain gauge for providing a changing chest measurement as described above, whereby the strain gauge measures the perimeter of the person's chest. Beneficially, inertial sensors and/or strain gauges are comparatively compact, cheep and precise. Therefore, a very unobtrusive and low-cost breathing detector can be provided.

Preferably, the force sensor comprises a pressure sensor. Advantageously, the breathing pattern of the user is also precisely and interference-free detectable with pressure-sensitive sensors which measures a mechanical force per area. The breathing of the user leads to a uniformly changing chest measurement which can be observed by the pressure-sensitive sensor. Consequently, the sensor is preferably attached near the chest of the user. In a preferred embodiment of the present invention the pressure-sensitive sensor comprises a piezoelectric sensor and in particular a piezoelectric sheet, which is provided in the bed and in the mattress respectively. It is an advantage of the present invention, that the breathing detector is not attached to the user. Beneficially, the breathing detector is rather invisible for the user.

Another object of the present invention is a breathing feedback device comprising:
- a breathing detector for detecting a breathing pattern,
- a processing unit for generating an output signal depending on the detected breathing pattern and
- a sound generator for playing the output signal,
- whereas the breathing detector comprises a heart rate detector.

As the heart rate of a human being gets modulated and determined by the breathing rate, the detection of the person's heart rate can be used determining the person's breathing pattern. This phenomenon is based on Respiratory sinus arrhythmia. Beneficially, measuring the heart rate is comparatively simple and can be realized with commercial and cost-effective hard rate monitors and pulse monitors.

Another object of the present invention is a breathing feedback device comprising:
- a breathing detector for detecting a breathing pattern,
- a processing unit for generating an output signal depending on the detected breathing pattern and
- a sound generator for playing the output signal,
- whereas the breathing detector comprises a heat-sensitive sensor.

Beneficially, the heat-sensitive sensor detects differences in temperature of the airflow into and/or out of the mouth/nose of the user during breathing. As mentioned above, the detected differences in temperature provides the determination of the actual breathing status. That means, that the heat-sensitive sensor detects whether the breathing user is currently breathing in or breathing out. As the airflow is inseparably connected with the breathing of the user, also the usage of a heat-sensitive sensor allows a very reliable and precise detection of the breathing pattern. In a preferred embodiment of the present invention the heat-sensitive sensor comprises a thermistor, which is provided in the airflow of the user. The thermistor is placed just under the nose of the user, for instance. In a preferred embodiment of the breathing feedback device according to one of the above mentioned objects of the present invention the breathing detector transmits the detected breathing pattern, in particular via a wireless connection, like infrared, Bluetooth, WLAN or the like, to the processing unit. Alternatively, the processing unit could be integrated into the breathing detector or into the sound generator. Also the breathing detector and the sound generator can be arranged in a common housing.

The processing unit preferably analyses the breathing pattern and transforms it into an output signal. Preferably, the breathing pattern is analyzed to determine the breathing characteristics, like the frequency, the volume and/or the tone color. Also a comparison of the detected breathing pattern with a stored breathing pattern is conceivable, whereby the breathing person is identified by the breathing feedback device in dependence of the comparison and its individual processing parameters are initiated, for instance. Furthermore, a supervision of the healthfulness of the user on the basis of the breathing pattern is possible.

In the simplest case the processing unit generates an output signal which is mainly similar to the breathing pattern. In a preferred embodiment the output signal and in particular the frequency, the volume, the tone, the tone color and likewise processing parameters of the output signal are adjusted depending on the detected breathing pattern. The output signal is mainly similar to the breathing pattern, but with a lower volume, for instance.

In another preferred embodiment the output signal is modulated in some way another sound which is fed back to the user by the sound generator. For example, one can make the sound of a wave on the beach which can be a noise signal filtered with a band-pass filter with a rising center frequency. Conceivably are also nature sounds, water sounds, music, speech, uniform tons or melodies and the like. As soon the breathing rate is below a certain threshold, preferably the output signal might be faded out or switched off in order not to disturb the user, or even awake the user as he has fallen asleep. Also an adjustable timer for stopping the output signal is conceivable.

The output signal is played by the sound generator to audibly feedback the output signal to the user, whereby the sound generator preferably comprises an amplifier, one or multiple loudspeakers, headphones and/or earphones. The sound generator can be mounted everywhere, but the preferred location would be above the bed and/or near the head of the user, so that the sound beam can easily be directed.

In a preferred embodiment of the breathing feedback device the breathing feedback device comprises an output unit for generating visual and/or tactile signals depending on the output signal. Advantageously, the user gets an additional visual and/or tactile feedback of his breathing behavior. The feedback can be a little vibration in the mattress or a cushion, for instance. Alternatively, the output unit comprises a lamp, which intensity is modulated with the breathing rate. The lamp might be spatially modulated with e.g. a little array of LED's.

In another preferred embodiment of the breathing feedback device according to one of the above mentioned objects of the present invention the breathing feedback device comprises multiple breathing detectors, multiple processing units and/or multiple sound generators. The breathing feedback device consists of a double implementation of its principle to function in situations in which a double bed is present or twin beds are present, for example. Either user can then have his or hers own breathing detector and distinguishable feedback sound. The user can adjust and configure the processing unit by a user-interface.

Another object of the present invention is a method for operating a breathing feedback device comprising the steps of:
- detecting a breathing pattern,
- generating an output signal depending on the detected breathing pattern and
- playing the output signal.

As described above, the step of detecting the breathing pattern beneficially comprises the usage of a camera, a heat-sensitive sensor, a pressure-sensitive sensor, an inertial sensor and/or a heart rate detector to increase the accuracy, the dependability and the cost-efficiency of said method in comparison to the prior art.

Preferably, the step of generating an output signal includes a step of analyzing the breathing pattern, wherein the coherence of the detected breathing pattern is verified. If the detected breathing pattern deviates from typical uniform and monotonous breathing characteristics, because the user is speaking for instance, the generation or playing of the output signal is stopped immediately. Furthermore, the frequency of the breathing pattern is preferably supervised and if the frequency falls below a certain threshold, because the user falls asleep for instance, the generation and/or playing of the output signal is also stopped or faded out. In another preferred embedment the detected breathing pattern is compared with a stored breathing pattern to identify a certain user and consequently setup certain output signal characteristics, for instance.

Particularly, the breathing feedback device is used as a general aid for improving breathing. More specifically, it can be used as an aid for falling asleep. Alternatively, making noise audible can have a direct, very relaxing and quietening influence on the person who's breathe is being monitored and on other persons present (e.g. a worrying partner).

These and other characteristics, features and advantages of the present invention will become apparent from the following detailed description, taken in conjunction with the accompanying drawing, which illustrates, by way of example, the principles of the invention. The description is given for the sake of example only, without limiting the scope of the invention. The reference figures quoted below refer to the attached drawing.

### BRIEF DESCRIPTION OF THE DRAWINGS

- Figure 1: shows a breathing feedback device according to a first embodiment of the present invention and
- Figure 2: shows a breathing feedback device according to a second embodiment of the present invention.

### DETAILED DESCRIPTION OF EMBODIMENTS

The present invention will be described with respect to particular embodiments and with reference to a certain drawing but the invention is not limited thereto but only by the claims. The drawing described is only schematic and is non-limiting. In the drawing, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated. Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein. It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

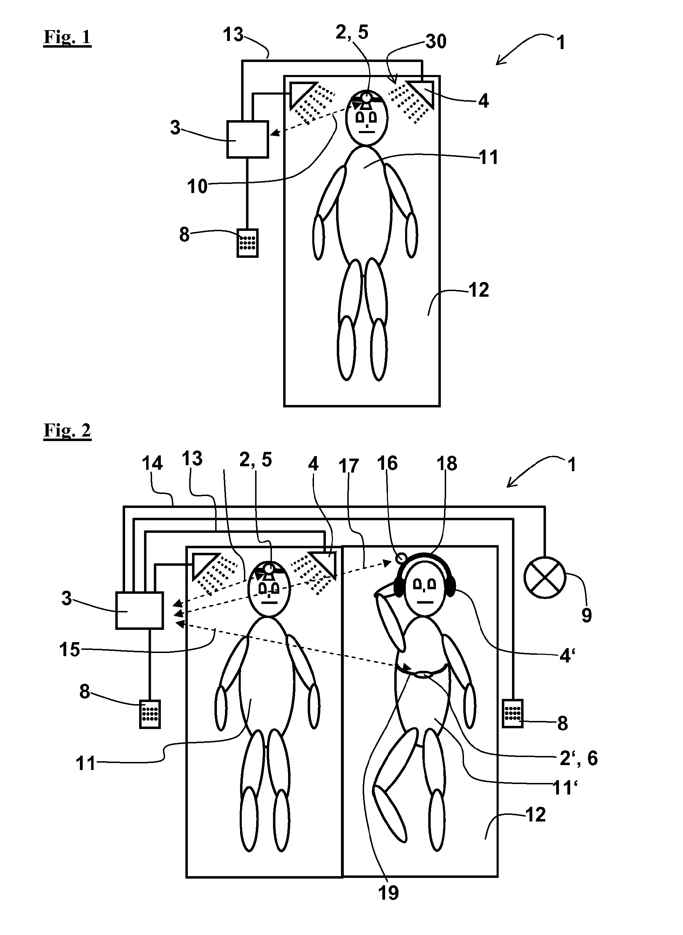

Figure 1 illustrates a breathing feedback device 1 according to a first exemplary embodiment of the present invention. The breathing feedback device 1 comprises a breathing detector 2 which is attached to the head of a user 11 by means of a headband or the like, whereas the user 11 relaxes or sleeps on a mattress 12. The breathing detector 2 comprises a thermographic camera 5 observing the airflow into and out of the mouth of the user 11 and determining a breathing pattern of the user 11 based on the thermal characteristics and the content of moisture of the airflow. The breathing pattern is transmitted to a processing unit 3 via a wireless connection 10. The processing unit 3 analyses the detected breathing pattern and transforms it into an output signal 30 which depends upon the characteristics of the breathing pattern. Furthermore, the processing unit 3 is hardwired 13 to sound generators 4, which feature two loudspeakers provided near the head of the user 11. The loudspeakers play the output signal 30 giving the user 11 an audible feedback concerning his breathing behavior. The transformation of the processing unit 3 and respectively the characteristics of the output signal 30 are adjustable by the user 11 through a user-interface 8 which is connected to the processing unit 3. In the simplest case the processing unit 3 generates an output signal 30 which is mainly similar to the breathing pattern. Although it is possible for the user 11 to adjust the processing unit 3 in such a manner, that the output signal 30 and in particular the frequency, the volume, the tone, the tone color and likewise characteristics of the output signal 30 are adjusted in dependency of the detected breathing pattern. For example, the output signal 30 is mainly similar to the breathing pattern, but with a lower volume. In another adjustable configuration of the processing unit 3 the output signal 30 is modulated in some way another sound which is fed back to the user 11 by the sound generator 4. For example, one can make the sound of a wave on the beach which can be a noise signal filtered with a band-pass filter with a rising center frequency. Conceivably are also nature sounds, water sounds, music, speech, uniform tons or melodies and the like.

Figure 2 shows a breathing feedback device 1 according to a second embodiment of the present invention, which is quite similar to the first embodiment illustrated in figure 1, whereas the breathing feedback device 1 according to the second embodiment comprises an additional breathing detector 2'. The additional breathing detector 2' features a force sensor 6 and in particular an inertial sensor. The inertial sensor, which is attached to a further user 11' with a chest strap 19, measures the breathing of the further user 11' in dependency of the motion of his chest and transmits 15 the detected further breathing pattern to the processing unit 3. The processing unit 3 generates an additional output signal in dependency of the further breathing pattern and transmits the additional output signal to a further sound generator 4' via wireless connection 17. The further sound generator 4' comprises headphones 18 with a receiving unit 16 receiving the additional output signal, whereas the headphones 18 are attached to the head of the further user 11'. The breathing feedback device 1 features an additional user-interface 8 which is operable by the further user 11'. Furthermore, the breathing feedback device 1 comprises a lamp 9 which is hardwired 14 to the processing unit 3 and which intensity is modulated with the breathing rate and respectively with the (further) output signal.

## Claims

1. A breathing feedback device (1) comprising:
- a breathing detector (2, 2') for detecting a breathing pattern,
- a processing unit (3) for generating an output signal (30) depending on the detected breathing pattern and
- a sound generator (4, 4') for playing the output signal (30),
- whereas the breathing detector (2, 2') comprises a camera (5).

2. A breathing feedback device (1) according to claim 1, **characterized in that** the camera (5) comprises an optical camera.

3. A breathing feedback device (1) according to claim 1, **characterized in that** the camera (5) comprises a thermographic camera.

4. A breathing feedback device (1) according to claim 1, **characterized in that** the camera (5) comprises a radar detection device.

5. A breathing feedback device (1) according to claim 1, **characterized in that** the camera (5) comprises a Photoplethysmographic Imager.

6. A breathing feedback (1) device comprising:
- a breathing detector (2, 2') for detecting a breathing pattern,
- a processing unit (3) for generating an output signal (30) depending on the detected breathing pattern and
- a sound generator for playing the output signal (30),
- whereas the breathing detector (2, 2') comprises a force sensor (6).

7. A breathing feedback device (1) according to claim 6, **characterized in that** the force sensor comprises an inertial sensor.

8. A breathing feedback device (1) according to claim 6, **characterized in that** the force sensor comprises a pressure sensor.

9. A breathing feedback device (1) according to claim 6, **characterized in that** the force sensor comprises a strain gauge.

10. A breathing feedback device (1) comprising:
- a breathing detector (2, 2') for detecting a breathing pattern,
- a processing unit (3) for generating an output signal (30) depending on the detected breathing pattern and
- a sound generator (4, 4') for playing the output signal (30),
- whereas the breathing detector (2, 2') comprises a heart rate detector.

11. A breathing feedback device (1) according to one of the preceding claims, **characterized in that** the processing unit (3) comprises means for adapting the volume, the frequency, the amplitude, the tone and/or the tone color of the output signal (30) and/or a user-interface (8).

12. A breathing feedback device (1) according to one of the preceding claims, **characterized in that** the breathing feedback device (1) comprises an output unit (9) for generating visual and/or tactile signals depending on the output signal 30).

13. A breathing feedback device (1) according to one of the preceding claims, **characterized in that** the breathing feedback device (1) comprises multiple breathing detectors, multiple processing units and/or multiple sound generators.

14. Method for operating a breathing feedback device (1) according to one of the preceding claims comprising the steps of:
- detecting a breathing pattern,
- generating an output signal (30) depending on the detected breathing pattern and
- playing the output signal (30).

15. Method according to claim 14, **characterized in that** the step of generating an output signal (30) includes a step of analyzing the breathing pattern and/or a step of comparing the breathing pattern with a stored breathing pattern.
